(19) **Europäisches Patentamt / European Patent Office / Office européen des brevets**

(11) **EP 4 458 989 B1**

(12) **EUROPEAN PATENT SPECIFICATION**

(45) Date of publication and mention of the grant of the patent:
**18.02.2026 Bulletin 2026/08**

(21) Application number: **23189193.8**

(22) Date of filing: **02.08.2023**

(51) International Patent Classification (IPC):
**C12Q 1/6886** (2018.01)

(52) Cooperative Patent Classification (CPC):
**C12Q 1/6886**; C12Q 2600/154

(54) **KIT FOR IDENTIFYING TUMOR TISSUE-OF-ORIGIN AND DATA ANALYSIS METHOD**

KIT ZUR IDENTIFIZIERUNG VON TUMORGEWEBE DES URSPRUNGS UND DATENANALYSEVERFAHREN

KIT D'IDENTIFICATION DE TISSU D'ORIGINE TUMORALE ET PROCÉDÉ D'ANALYSE DE DONNÉES

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **04.05.2023 CN 202310501572**

(43) Date of publication of application:
**06.11.2024 Bulletin 2024/45**

(73) Proprietor: **Hangzhou ShengTing Medical Technology Ltd.**
**Hangzhou Zhejiang 311113 (CN)**

(72) Inventors:
• GU, Hongcang
Hangzhou, 311113 (CN)
• WANG, Yunfei
Hangzhou, 311113 (CN)
• CHE, Xianrong
Hangzhou, 311113 (CN)
• MENG, Xianghe
Hangzhou, 311113 (CN)
• XIE, Changxiao
Hangzhou, 311113 (CN)
• XIE, Qiongwan
Hangzhou, 311113 (CN)
• WANG, Wenjun
Hangzhou, 311113 (CN)

(74) Representative: **Patent 42**
**5, rue Dicks**
**4081 Esch-zur-Alzette (LU)**

(56) References cited:
EP-A1- 2 898 100    WO-A1-2022/159035
WO-A2-2008/015396    CN-A- 113 249 439
CN-A- 113 604 540    US-A1- 2023 126 920

• GUO SHICHENG ET AL: "Identification of methylation haplotype blocks aids in deconvolution of heterogeneous tissue samples and tumor tissue-of-origin mapping from plasma DNA", vol. 49, no. 4, 6 March 2017 (2017-03-06), New York, pages 635 - 642, XP093043427, ISSN: 1061-4036, Retrieved from the Internet <URL:http://www.nature.com/articles/ng.3805> DOI: 10.1038/ng.3805

Remarks:
The complete document including Reference Table(s) and the Sequence Listing(s) can be downloaded from the EPO website

**Description**

**TECHNICAL FIELD**

The present disclosure relates to the technical field of biomedicine, in particular to a kit for identifying tumor tissue-of-origin and a data analysis method for a kit for identifying tumor tissue-of-origin.

**BACKGROUND**

Cancer metastasis refers to a process in which tumor cells fall off from a primary lesion, enter a circulatory system, transfer to other parts of the body, and continue to grow. The original tumor is called "primary lesion", and the newly formed tumor is called "secondary lesion" or "metastatic lesion". According to the stage of tumor cells entering the circulatory system, cancer metastasis can be divided into early dissemination model and late dissemination model. A difference between the two models lies primarily in an environment in which metastatic cancer cells mutate. In the late dissemination model, cancer cells mutate and proliferate at the primary lesion, and then metastasize through the circulatory system. At this time, the metastatic tumor has a high degree of genetic similarity with the primary tumor, and can be treated with same treatment methods as the primary tumor. In the early dissemination model, cancer cells mutate after passing through the circulatory system to reach their destination. In this model, the metastatic tumor shows a low genetic similarity with that of the cancer cells in the primary tumor, making it difficult to determine the location of the primary tumor.

When the tumor has developed into a real cancer, namely invasive cancer, it means that the cancer cells have invaded and infiltrated deeper from the place where they occurred. For invasive cancer, the metastasis advantage of cancer cells is the main cause of cancer death.

According to statistics, 90% of cancer deaths are caused by metastasis. Metastatic cancer is extremely difficult to treat. The attachment ability of cancer cells in patients is reduced or even completely lost, and the migration ability of cancer cells is enhanced, leading to the transfer of cancer cells from the original site to other body parts along with the blood or lymphatic system, thus finally forming new tumors. Tumor metastasis refers to a process in which tumor cells migrate from the original site to other body parts and continue to grow by invading the circulatory system.

Cancer of unknown primary (CUP) is a type of tumor that is diagnosed as metastatic, but has a primary location undetermined even after a comprehensive diagnostic workup. The CUP accounts for about 3% to 9% of the total number of malignant tumors, and shows a mortality rate ranking fourth or fifth among all cancers. The reason why a primary tumor cannot be determined may be that the primary tumor is too small, has been destroyed by the human immune system, or has been surgically removed. Patients with CUP generally have a poor prognosis. A median survival time after chemotherapy is 4 to 12 months, a 1-year survival rate is about 50%, and a 5-year survival rate is about 10%. Approximately 15-20% of CUP patients who receive site-specific chemotherapy have improved overall survival compared to patients treated with empiric chemotherapy.

Positron emission tomography/computed tomography (PET/CT), as once the most effective medical imaging tool to identify the primary location of CUP, was capable of diagnosing about 30% of CUP primary locations. Moller et al. detected the primary location of CUP using FDG-PET-CT. However, when the primary tumor has a small size, the PET/CT plays a limited role in tracing the origin of cancer.

Immunohistochemistry (IHC) with antibodies against tumor antigens has been the "gold standard" for the past two decades. Yet, the challenges remain: hand-picked antibody panels are primarily subjective, and the IHC analysis can identify primary sites in only 50-65% of patients with metastases and an even lower rate of 20-25% in CUP patients. In recent years, with the development of biotechnology, researchers have been able to detect the gene expression, miRNA, lncRNA, and methylation levels of tumor tissue cells, and obtain tissue-specific tumor characteristics through analysis. The molecular expression profiles of tumor cells in metastases are more similar to those in the primary tumor, but different from those in the metastatic site. This suggests that tumor origin can be traced based on the molecular expression profiles of metastatic tumor cells. At present, studies have constructed tumor traceability models, and achieved high accuracy by analyzing mRNA, miRNA, or lncRNA levels. Compared to the single-stranded RNAs, the double-stranded nature and the absence of a reactive 2'-hydroxyl group on the pentose ring make DNA more attractive for genetic testing. DNA methylation, the addition of a methyl group to the cytosine almost exclusively in the context of CpG dinucleotides, shows both cell- and tissue-specific patterns in the human genome, which can be used to determine the primary location of the tumor. Machine learning algorithms can discover patterns from a large amount of methylation data, and classify the tumor tissue origin accordingly. As a result, the machine learning algorithms are suitable for the methylation-based tumor tracing.

CN113249439 A (HANGZHOU SHENGTIANG MEDICAL TECH CO LTD, August 13, 2021) discloses library construction related adapter bottom 4 strand/linker. CN 113 604 540 A (HANGZHOU SHENGTING MEDICAL TECH CO LTD, November 5, 2021) discloses the RRBS library construction related methylated linker DNA (RRBS-4). WO 2008/015396 A2 (SOLEXA LTD [GB]; EARNSHAW DAVID JAMES [GB] ET AL., February 7, 2008) discloses the human BAC DNA clone amplifying PCR primer. EP 2 898 100 A1 (UNIV HONG KONG CHINESE [CN], July 29, 2015) discloses the use of methylation libraries. WO 2022/159035 A1 (NAT UNIV SINGAPORE [SG]; SINGAPORE HEALTH SERV PTE LTD [SG], July 28, 2022) and US 2023/126920 A1 (CI WEIMIN [US] ET AL, April 27, 2023) disclose use of FastQC to check the quality of the pair-end read generated by a MiSeq sequencer. After adapter trimming using Cutadapt Software, the reads were aligned to the hg38 human genome using Bismark Software. The aligned reads were deduplicated using Picard tools, following which the Bismark methylation extractor was used to obtain per-base methylation status of each fragment. GUO SHICHENG et al. (Identification of methylation haplotype blocks aids in deconvolution of heterogeneous tissue samples and tumor tissue-of-origin mapping from plasma DNA, NATURE GENETICS, March 6, 2017, pages 635-64, ISSN:1061-4036, DOI:10.1038/ng.3805) disclose that epigenetic information embedded in cfDNA has the potential for predicting a tumour tissue of origin.

## SUMMARY

In order to overcome the low accuracy and effectiveness of IHC marker recognition in the prior part, the present disclosure designs a novel DNA methylation-based technology, including methylated adapters with inline barcodes and matched PCR amplification primers. The technology has higher accuracy and effectiveness.

To achieve the above objective, the present disclosure adopts the following technical solutions: the present disclosure provides a kit for identifying tumor tissue-of-origin, including a serial methylated adapter, PCR amplification primers and a PCR amplification reagent, where the adapter consists of the nucleotide sequences of A01-T, A01-B, A02-T, A02-B, A03-T, A03-B, A04-T, A04-B, A05-T, A05-B, A06-T, and A06-B; and the PCR amplification primers consist of the nucleotide sequences of R01-F, R01-R, R02-F, and R02-R.

Preferably, the kit for identifying tumor tissue-of-origin further includes a PCR amplification reagent, where the PCR amplification reagent includes a polymerase, dNTP, $MgCl_2$, and Tris-HCl.

Preferably, the kit for identifying tumor tissue-of-origin further includes a dephosphorylase and a $10\times$ buffer selected from the group consisting of KAc, Tris-Ac, and $Mg(Ac)_2$. The buffer works well for multiple enzymatic reactions employed in this kit.

Preferably, the kit for identifying tumor tissue-of-origin further includes a dNTP mixture, where the dNTP mixture includes dATP, dCTP, and dGTP and excludes dTTP.

Preferably, the kit for identifying tumor tissue-of-origin further includes an end repair enzyme, a ligase, and ATP.

Preferably, the kit for identifying tumor tissue-of-origin further includes a negative control and a positive control, where the negative control is a healthy human blood leukocyte DNA, and the positive control is a cancer tissue sample DNA.

The present disclosure further provides a computer-implemented data analysis method for analyzing the data obtained by the kit for identifying tumor tissue-of-origin, including the following steps:

1) data preprocessing: conducting quality control on a raw off-machine data, and removing an adapter sequence and an inline barcode sequence of a raw data to obtain clean data;
2) alignment: allowing the clean data to be aligned to the human reference genome, and converting a resulting bam file generated by the alignment into an mHap file;
3) CpG methylation information statistics: extracting a methylation information of each CpG site;
4) quality control: removing a sample with less than 800,000 CpG sites and having a bisulfite conversion rate of less than 99%, an alignment rate of less than 50%, and a coverage of not less than $10\times$; and
5) analysis: analyzing a Beta value-based DNA methyaltion index, predicting all samples in sequence with a training set model, and outputting a probability value of each sample on 10 cancer types.

Preferably, the quality control is conducted on the raw off-machine data by a FastQC software component in step 1).

Preferably, the adapter sequence and the inline barcode sequence of the raw data are removed by Trim Galore software in step 1).

The beneficial effects of the present disclosure are: (1) The present disclosure is based on two methodological patents including "CN113604540A, Method for Quickly Constructing RRBS Sequencing Library Using Circulating Tumor DNA" and "CN113550013A, Method for Rapidly Constructing RRBS Sequencing Library Using Formalin-fixed Paraffin-Embedded Sample". In the present disclosure, a kit is developed for qualitatively detecting a methylation profile of a human genome in formalin-fixed paraffin-embedded (FFPE) tumor tissues or blood samples of patients initially diagnosed as cancer by clinicians. The kit has higher accuracy and effectiveness. The present disclosure solves the current problem that the primary tumor of some tumor patients cannot be determined by IHC. (2) The present disclosure further improves the prediction accuracy of tumor primary site through a matching analysis system.

## BRIEF DESCRIPTION OF THE DRAWINGS

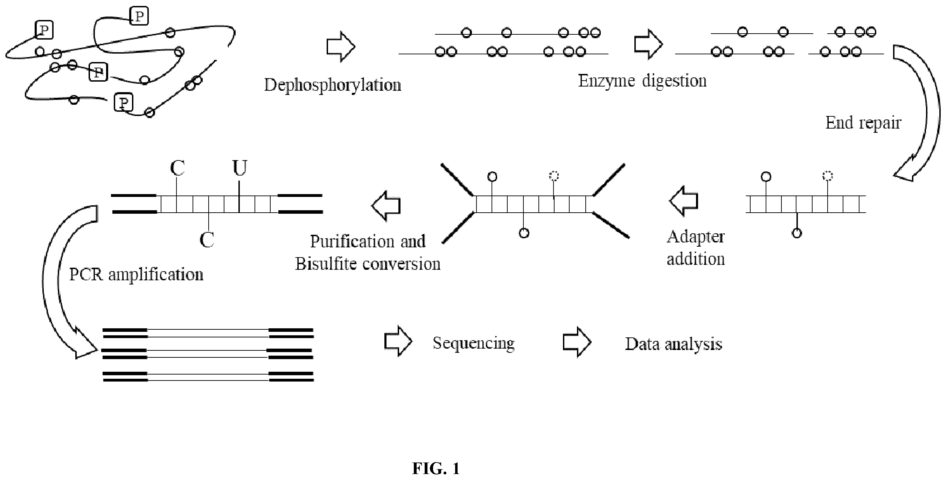

FIG. 1 shows a flowchart of the methylation detection of the present disclosure;

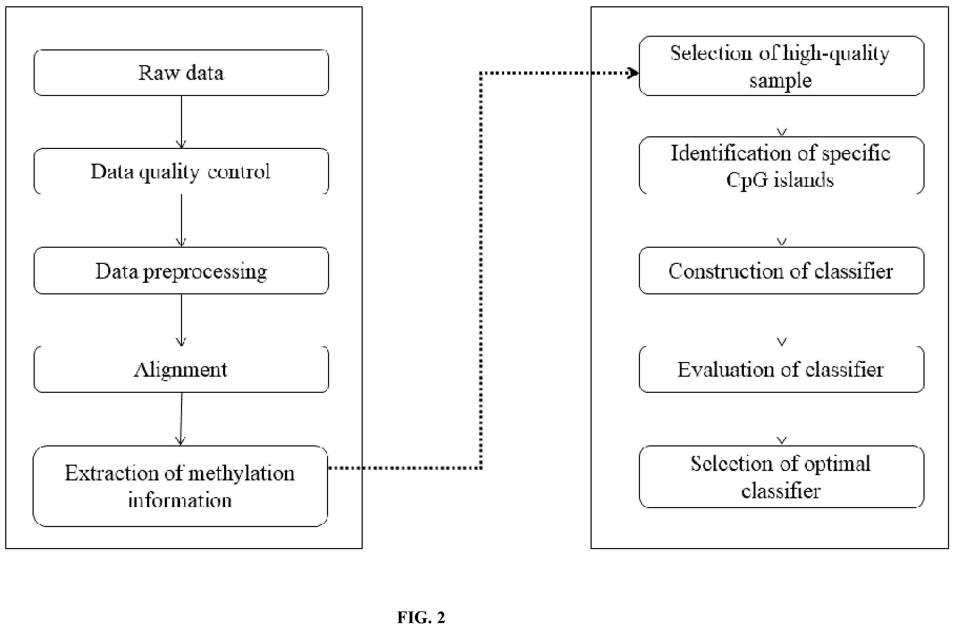

FIG. 2 shows a flowchart of constructing an analysis model of the present disclosure; and

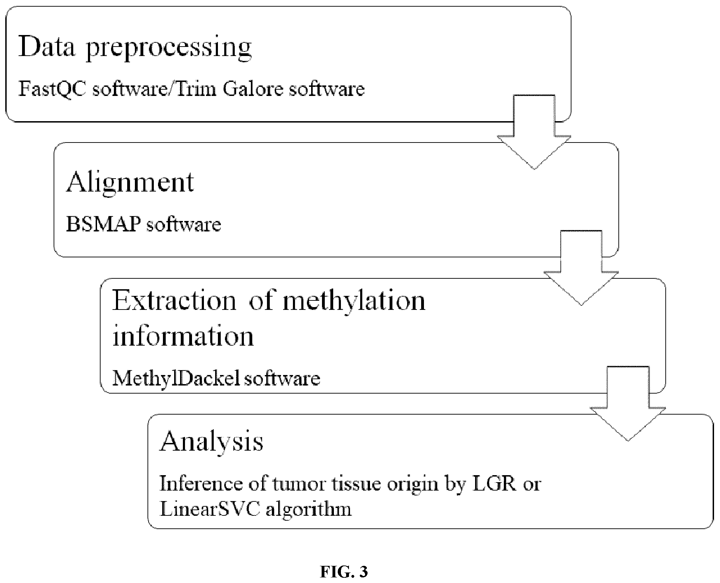

FIG. 3 shows a flowchart of the methylation analysis of the present disclosure.

## DETAILED DESCRIPTION OF THE EMBODIMENTS

The following further describes the present disclosure in detail with reference to the accompanying drawings and examples.

In one example, the present disclosure provides a kit for identifying tumor tissue-of-origin, including an adapter, PCR amplification primers, PCR amplification reagents, a dephosphorylase, a 10× buffer, a dNTP mixture, an end repair enzyme, a ligase, ATP, a negative control, and a positive control, where the adapter includes nucleotide sequences of A01-T, A01-B, A02-T, A02-B, A03-T, A03-B, A04-T, A04-B, A05-T, A05-B, A06-T, and A06-B. The PCR amplification reagent includes a polymerase, dNTP, $MgCl_2$, and Tris-HCl. The 10× buffer is selected from the group consisting of KAc, Tris-Ac, and $Mg(Ac)_2$.

Adapter nucleotide sequences are specifically as follows:

| | |
|---|---|
| A01-T | mCTmCAmCGAmCGmCTmCTmCmCGTmCTAmCAAmCmC-S-T (SEQ ID NO: 1) |
| A01-B | Pho-GGTTGTAGATmCGGAAGAGmCAmCAmCGTmCTAAmC (SEQ ID NO: 2) |
| A02-T | mCTAmCAmCGmCGmCTmCTmCmCGATmCTAmCTmCAmC-S-T (SEQ ID NO: 3) |
| A02-B | Pho-GTGAGTAGATmCGGAAGAGmCAmCAmCGTmCTGAAmC (SEQ ID NO: 4) |
| A03-T | mCTAmCAmCGmCGmCTmCTmCmCGATmCTAGGATG-S-T (SEQ ID NO: 5) |
| A03-B | Pho-mCATmCmCTAGATmCGGAAGAGmCAmCAmCGTmCTGAAmC (SEQ ID NO: 6) |
| A04-T | mCTAmCAmCAmCGmCTmCTTmCmCGATmCTATmCGAmC-S-T (SEQ ID NO: 7) |
| A04-B | Pho-GTmCGATAGATmCGGAAGAGmCAmCAmCGTmCTGAAmC (SEQ ID NO: 8) |
| A05-T | mCTAmCAmCGAmCGmCTmCTTmCmCGTmCTmCAAGAG-S-T (SEQ ID NO: 9) |
| A05-B | Pho-mCTmCTTGAGATmCGGAAGAGmCAmCAmCGTmCTGAAmC (SEQ ID NO: 10) |
| A06-T | mCTAmCAmCGAmCGmCTmCTTmCmCGATmCTmCATAmC-S-T (SEQ ID NO: 11) |
| A06-B | Pho-GTmCATGAGATmCGGAAGAGmCAmCAmCmCTGAAmC (SEQ ID NO: 12) |

The PCR amplification primers include nucleotide sequences of R01-F, R01-R, R02-F, and R02-R. The primer nucleotide sequences of the PCR amplification primers are specifically as follows:

| | |
|---|---|
| R01-F | AATGATACGGGCGACCACCGCACTCTTTCCCTACACGACGCTCTTCCGATCT (SEQ ID NO: 13) |
| R01-R | CAAGCAGAAGACGGCATACGAGATTCGCCTTAGTGAGTTCAGACGTGTGCTCTTCCGATCT (SEQ ID NO: 14) |
| R02-F | AATGATACGGGCGACCACCGATACACTCTTTCCCTACACGAGGCTCTTCCGATCT (SEQ ID NO: 15) |
| R02-R | CAAGCAGAAGACGGCATACGAGATCTAGTACGGGTGTCAGACGTGTGCTCTTCCGATCT (SEQ ID NO: 16) |

In another example, the present disclosure provides a data analysis method for a kit for identifying tumor tissue-of-origin, including the following steps:

1) data preprocessing: conducting quality control on a raw off-machine data, and removing an adapter sequence and an inline barcode sequence of a raw data to obtain clean data; the quality control is conducted on the raw off-machine data by a FastQC software component in step 1); the adapter sequence and the inline barcode sequence of the raw off-machine data are removed by Trim Galore software in step 1);
2) alignment: allowing the clean data to be aligned to the human reference genome, and converting a resulting bam file generated by the alignment into an mHap file;
3) CpG methylation information statistics: extracting a methylation information of each CpG site;
4) quality control: removing a sample with less than 800,000 CpG sites and having a bisulfite conversion rate of less than 99%, an alignment rate of less than 50%, and a coverage of not less than 10×; and
5) analysis: analyzing a Beta value-based methylation index, predicting all samples in sequence with a training set model, and outputting a probability value of each sample on 10 cancer types.

**Example 1:** a detection process of this product was as follows:

1) DNA dephosphorylation treatment:

A DNA dephosphorylation system included: 5 ng to 500 ng of DNA, 0.1 $\mu$L to 1.0 $\mu$L of dephosphorylase, 0.3 $\mu$L to 3.0 $\mu$L of buffer, and adding water to a total volume of 10 $\mu$L to 50 $\mu$L. The DNA dephosphorylation included: treatment at 37°C for 5 min to 50 min, and treatment at 75°C for 5 min to 30 min.

2) DNA digestion:

A DNA digestion system included: 0.1 $\mu$L to 1.0 $\mu$L of endonuclease and 0.1 $\mu$L to 1.0 $\mu$L of buffer were added to the DNA dephosphorylation treatment system. DNA digestion included: treatment at 37°C for 10 min to 100 min, and treatment at 70°C for 5 min to 30 min.

3) End repair of the library:

An end repair system included: 0.1 $\mu$L to 1.0 $\mu$L of end repair enzyme, 0.1 $\mu$L to 1.0 $\mu$L of buffer, and 0.1 $\mu$L to 1.0 $\mu$L of dNTP mixture were added to the DNA digestion system. End repair reaction included: treatment at 30°C for 10 min to 30 min, treatment at 37°C for 10 min to 30 min, treatment at 70°C for 5 min to 30 min.

4) Adapter ligation:

An adapter ligation system included: 0.1 $\mu$L to 1.0 $\mu$L of ATP, 0.4 $\mu$L to 4.0 $\mu$L of ligase, 0.5 $\mu$L to 5.0 $\mu$L of buffer, and 0.3 $\mu$L to 3.0 $\mu$L of adapter were added to the library end repair system. An adapter ligation procedures included: treatment at 16°C for 1 h to 4 h, treatment at 70°C for 10 min to 30 min.

5) Nucleic acid purification:

The system was purified with a nucleic acid purification reagent, and 40 $\mu$L to 60 $\mu$L of a DNA solution was taken into a transformation reaction.

6) Bisulfite conversion:

The purified nucleic acid was subjected to sodium bisulfite conversion with a conversion kit.

7) PCR amplification:

A PCR amplification system included: 20 $\mu$L to 30 $\mu$L of transformed DNA, 10 $\mu$L to 25 $\mu$L of PCR amplification reagent, and 1 $\mu$L to 5 $\mu$L of PCR amplification primers.
PCR amplification program (as shown in the table below):

| Temperature | Time | Number of cycles |
|---|---|---|
| 98°C | 45 sec | 1 |
| 98°C | 5 sec | 6 |
| 58°C | 5 sec | |
| 72°C | 10 sec | |
| 98°C | 5 sec | 14 |
| 65°C | 5 sec | |
| 72°C | 10 sec | |
| 72°C | 5 min | 1 |
| 4°C | hold | |

8) Library purification:

The library was purified with a nucleic acid purification reagent, and 10 μL to 50 μL of the DNA solution was stored in a 1.5 mL EP tube.

9) Library quality control:

Quality control of library concentration: Qubit® dsDNA HS Assay Kit was used for quantifying a library concentration, and the library concentration should be greater than 2.5 ng/μL. Quality control of library fragment length: Agilent High Sensitivity DNA Kit was used for quality inspection of library fragments. If the size distribution of the library DNA was between 200 bp to 400 bp, the library was considered as qualified.

10) Convention of general library into MGI library (Illumina platform skipped this step):

A universal library was converted into an MGI library with an MGIEasy Universal Library Conversion Kit.

11) Sequencing:

Sequencing was conducted with the matching sequencing reagents of a gene sequencer MGISEQ-2000 or the matching sequencing reagents of an Illumina sequencer.

A data analysis method for the kit for identifying tumor tissue-of-origin included:

1) Data preprocessing: quality control was conducted on a raw off-machine data with a FastQC software component, and an adapter sequence and an inline barcode sequence of a raw data were removed with Trim Galore software to obtain clean data.

2) Alignment: the clean data was aligned to the human reference genome (version hg19), and a resulting bam file generated by the alignment was converted into an mHap file.

3) CpG methylation information statistics: a methylation information of each CpG site was extracted with methylation data analysis software MethylDackel.

4) Quality control: to obtain high-quality samples, a sample with less than 800,000 CpG sites and having a bisulfite conversion rate of less than 99%, an alignment rate of less than 50%, and a coverage of not less than $10\times$ was removed.

5) Feature selection: CpG islands that met the conditions for subsequent analysis were selected. A specific screening method was as follows: the coverage of CpG islands should be greater than $100\times$. Moreover, potential CpG islands that could be used as biomarkers by t-test were selected from the DNA methylation data set of 498 primary tumors and 57 tumor-adjacent normal tissues. The selected CpG islands should exhibit methylation levels in one cancer type that were significantly different (FDR≤0.01) from those in other cancer types. Moreover, the methylation level should be significantly higher or lower than 57 tumor-adjacent normal tissues (FDR≤0.01).

In this example, 4 different methods were used to characterize the methylation level of CpG islands. The 4 methods were: mean methylation (Beta value), Proportion of Discordant Reads (PDR), Cell Heterogeneity-Adjusted cLonal Methylation (CHALM), and Methylated Haplotype Load (MHL). Each method could obtain a set of CpG islands, which were used for subsequent classifier construction.

6) Classifier construction: 28 classifiers were constructed using 7 machine learning algorithms (adaboost, KNN, LGR, LinearSVC, NB, RF, and SVM) and the above 4 sets of CpG islands, and then a validation set was used to evaluate these classifiers. The main indicators of evaluation included Precision, Recall, F1 score, and Accuracy. The calculation formulas were as follows:

$$Recall = TP/(TP + FN)$$

$$Precision = TP/(TP + FP)$$

$$F1 = 2(recall * precision)/(recall + precision)$$

$$Accuracy = (TP + TN)/(TP + FP + TN + FN)$$

The predicted AUC of the classifier constructed based on the above method (the table below)

| Methods | Beta | CHALM | MHL | PDR |
|---|---|---|---|---|
| adaboost | 0.88 | 0.56 | 0.88 | 0.52 |
| KNN | 0.81 | 0.58 | 0.80 | 0.54 |
| LGR | 0.95 | 0.58 | 0.92 | 0.60 |
| LinearSVC | 0.95 | 0.54 | 0.92 | 0.57 |
| NB | 0.58 | 0.53 | 0.55 | 0.54 |
| RF | 0.88 | 0.57 | 0.86 | 0.58 |
| SVM | 0.90 | 0.61 | 0.85 | 0.59 |

In this example, the LGR and LinearSVC algorithms could also be used to analyze the Beta value-based methylation index, showing the maximum AUC.

**Example 2:**

The produced human genome methylation detection kit was tested, the clinical samples from different hospitals were detected, and the clinicopathologic diagnosis method was used as a comparative verification method.

Comparative verification method - the clinicopathologic diagnosis method is based on the current medical level and is the result of a comprehensive determination of information by professional doctors, where the information included blood and biochemical tests, urinalysis, fecal occult blood test, radiological examination of the suspected tumor primary, pathological immunohistochemistry, and medical history.

A testing method of this kit included the following steps:

Paraffin-embedded pathological tissues: a sample was collected from the lesion tissues to confirm that there were at least 30% tumor-related lesion tissues. Paraffin-embedded pathological tissues or section samples were confirmed to carry neoplastic lesion cells. The samples that had been stored for not more than 1 year were selected, and DNA extraction was conducted with a commercial kit using not less than 8 pieces of 5 $\mu$m sections or no less than 5 pieces of 10 $\mu$m sections.

An effective DNA concentration was determined by Qubit® dsDNA HS Assay Kit. If the DNA concentration was not less than 2 ng/$\mu$L, and a total amount of DNA was not less than 50 ng, it was determined to be qualified.

1) DNA dephosphorylation treatment:

A DNA dephosphorylation system included: 50 ng of DNA, 1.0 $\mu$L of dephosphorylase, 10 $\mu$L of buffer, and adding water to a total volume of 30 $\mu$L. DNA dephosphorylation included: treatment at 37°C for 50 min, and treatment at 75°C for 20 min.

2) DNA digestion:

A DNA digestion system included: 1.0 $\mu$L of endonuclease and 0.2 $\mu$L of buffer were added to the DNA dephosphorylation treatment system. DNA digestion included: treatment at 37°C for 90 min, and treatment at 70°C for 10 min.

3) End repair of the library:

An end repair system included: 1.0 $\mu$L of end repair enzyme, 0.4 $\mu$L of buffer, and 0.8 $\mu$L of dNTP mixture were added to the DNA digestion system. End repair reaction included: treatment at 30°C for 25 min, treatment at 37°C for 25 min, treatment at 70°C for 10 min.

4) Adapter ligation:

An adapter ligation system included: 0.2 $\mu$L of ATP, 0.4 $\mu$L of ligase, 0.6 $\mu$L of buffer, and 3.0 $\mu$L of adapter were added to the library end repair system. An adapter ligation procedures included: treatment at 16°C for 4 h, treatment at 70°C for 15 min.

5) Nucleic acid purification:

The system was purified with a nucleic acid purification reagent, and 40 $\mu$L of a DNA solution was taken into a transformation reaction.

6) Bisulfite conversion:

The purified nucleic acid was subjected to sodium bisulfite conversion with a conversion kit.

7) PCR amplification:

A PCR amplification system included: 20 $\mu$L of transformed DNA, 25 $\mu$L of PCR amplification reagent, and 5 $\mu$L of PCR primers.
PCR amplification program (as shown in the table below):

| Temperature | Time | Number of cycles |
|---|---|---|
| 98°C | 45 sec | 1 |
| 98°C | 5 sec | 6 |
| 58°C | 5 sec | |
| 72°C | 10 sec | |
| 98°C | 5 sec | 14 |
| 65°C | 5 sec | |
| 72°C | 10 sec | |
| 72°C | 5 min | 1 |
| 4°C | hold | |

8) Library purification:

The library was purified with a nucleic acid purification reagent, and 35 $\mu$L of the DNA solution was stored in a 1.5 mL EP tube.

9) Library quality control:

Quality control of library concentration: Qubit® dsDNA HS Assay Kit was used for quality control of a library concentration, and the library concentration was greater than 2.5 ng/$\mu$L. Quality control of library fragment length: Agilent High Sensitivity DNA Kit was used for quality inspection of library fragments. If the size of the main band of the fragment was 200 bp to 400 bp, the library fragment was qualified.

10) Convention of general library into MGI library:

A universal library was converted into an MGI library with an MGIEasy Universal Library Conversion Kit.

11) Sequencing:

Sequencing was conducted with the matching sequencing reagents of a gene sequencer MGISEQ-2000.

**Example 3**

The present disclosure further provided a data analysis method for a kit for identifying tumor tissue-of-origin, including the following steps:

1) Data preprocessing: quality control was conducted on a raw off-machine data with a FastQC software component, and an adapter sequence and an inline barcode sequence of a raw data were removed with Trim Galore software to obtain clean data.
2) Alignment: the clean data was aligned to the human reference genome (version hg19), and a resulting bam file generated by the alignment was converted into an mHap file.
3) CpG methylation information statistics: a methylation information of each CpG site was extracted with methylation data analysis software MethylDackel.
4) Quality control: a sample with less than 800,000 CpG sites and having a bisulfite conversion rate of less than 99%, an alignment rate of less than 50%, and a coverage of not less than $10\times$ was removed.
5) Analysis: a Beta value-based methylation index was analyzed with LGR and LinearSVC algorithms, all samples were predicted in sequence with a training set model, and a probability value of each sample on 10 cancer types was output.
6) The consistency analysis results were shown in summary Table 1. The analysis showed the prediction results of the human genome methylation detection kit (combined with a probe-anchored polymerization sequencing method) on the primary site of cancer (according to the probability value, a first-ranked result was selected to be compared with a pathological diagnosis result), with an overall accordance rate of 78.75%. The above results had high total accordance rate and consistency with the clinical diagnosis results, thus preliminarily verifying that the kit showed high accuracy and effectiveness in detecting the primary site of cancer.

Table 1: Consistency analysis results (compared with results ranked first in probability value)

| SN | Cancer name | Sample size | Samples with accurate prediction | Accordance rate |
|---|---|---|---|---|
| 1 | Breast cancer | 27 | 24 | 88.89% |
| 2 | Lung cancer group | 27 | 23 | 85.19% |
| 3 | Gastric cancer | 30 | 20 | 66.67% |
| 4 | Intestinal cancer | 31 | 30 | 96.77% |
| 5 | Head and neck cancer | 22 | 15 | 68.18% |
| 6 | Thyroid cancer | 25 | 23 | 92.00% |
| 7 | Cervical cancer | 16 | 9 | 56.25% |
| 8 | ovarian cancer | 26 | 21 | 80.77% |
| 9 | Liver cancer | 23 | 18 | 78.26% |
| 10 | Esophagus cancer | 13 | 6 | 46.15% |
| Total | Total number of samples | 240 | 189 | 78.75% |

Considering that some cancers such as cervical cancer only occur in women, and when a difference between the first and second predicted probability values is small, it is possible that a corresponding part of the predicted probability value ranked second is the real primary cancer lesion. Therefore, the top two results of the predicted probability values were analyzed, and one of the top two consistent with the clinical diagnosis result was regarded to be consistent. The results were shown in the table. The accordance rate of cancer types including gastric cancer, head and neck cancer, cervical cancer, and esophagus cancer had been significantly improved, with an overall accuracy increased to 88.33% (Table 2).

Table 2: Consistency analysis results (compared with results ranked TOP2 in probability value)

| SN | Cancer name | Sample size | TOP2 prediction samples | Accuracy rate |
|---|---|---|---|---|
| 1 | Breast cancer | 27 | 25 | 92.59% |
| 2 | Lung cancer group | 27 | 26 | 96.30% |
| 3 | Gastric cancer | 30 | 22 | 73.33% |
| 4 | Intestinal cancer | 31 | 30 | 96.77% |
| 5 | Head and neck cancer | 22 | 20 | 90.91% |
| 6 | Thyroid cancer | 25 | 25 | 100.00% |
| 7 | Cervical cancer | 16 | 12 | 75.00% |
| 8 | ovarian cancer | 26 | 22 | 84.62% |
| 9 | Liver cancer | 23 | 20 | 86.96% |
| 10 | Esophagus cancer | 13 | 10 | 76.92% |
| Total | Total number of samples | 240 | 212 | 88.33% |

Among the 240 samples tested in this study, 89 carcinomas were known to be poorly or moderately differentiated. The results were shown in Table 3. Calculated according to the highest predicted probability value (TOP1), the accuracy could reach 75.28%. The accuracy was increased to 85.39% by including the top two predicted probability values (TOP2) in the calculation. This indicated that the ability of this product to determine the primary site of poorly differentiated cancer was better than that of immunohistochemistry reported by the current technical level.

Table 3 Consistency analysis results of moderately and poorly differentiated cancer tissues

| SN | Cancer name | Sample size | TOP1 prediction samples | Accuracy rate | TOP2 prediction samples | Accuracy rate |
|---|---|---|---|---|---|---|
| 1 | Breast cancer | N/A | N/A | N/A | N/A | N/A |
| 2 | Lung cancer group | 14 | 11 | 78.57% | 13 | 92.86% |
| 3 | Gastric cancer | 21 | 15 | 71.43% | 16 | 76.19% |
| 4 | Intestinal cancer | 17 | 16 | 94.12% | 16 | 94.12% |
| 5 | Head and neck cancer | 12 | 8 | 66.67% | 11 | 91.67% |
| 6 | Thyroid cancer | N/A | N/A | N/A | N/A | N/A |
| 7 | Cervical cancer | 6 | 3 | 50.00% | 3 | 50.00% |
| 8 | ovarian cancer | 9 | 9 | 100.00% | 9 | 100.00% |
| 9 | Liver cancer | 5 | 4 | 80.00% | 4 | 80.00% |
| 10 | Esophagus cancer | 5 | 1 | 20.00% | 4 | 80.00% |
| Total | | 89 | 67 | 75.28% | 76 | 85.39% |

In general, the determination result of this product had high total accordance rate and consistency with those of the clinical diagnosis results. This preliminarily verified that the human genome methylation detection kit had a clinical value for the detection of the primary site of cancer, and showed high accuracy and effectiveness.

## Claims

1. A kit for identifying tumor tissue-of-origin, comprising an adapter, PCR amplification primers and a PCR amplification reagent, wherein the adapter consists of the nucleotide sequences set forth in SEQ ID NOs: 1-12; and wherein the PCR amplification primers consist of the nucleotide sequences set forth in SEQ ID NOs: 13-16.

2. The kit for identifying tumor tissue-of-origin according to claim 1, further comprising a PCR amplification reagent, wherein the PCR amplification reagent comprises a polymerase, dNTP, $MgCl_2$, and Tris-HCl.

3. The kit for identifying tumor tissue-of-origin according to claim 1, further comprising a dephosphorylase and a $10\times$ buffer, wherein the $10\times$ buffer is selected from the group consisting of KAc, Tris-Ac, and $Mg(Ac)_2$.

4. The kit for identifying tumor tissue-of-origin according to claim 1, further comprising a dNTP mixture, wherein the dNTP mixture comprises dATP, dCTP, and dGTP.

5. The kit for identifying tumor tissue-of-origin according to claim 1 or 2 or 3 or 4, further comprising an end repair enzyme, a ligase, and ATP.

6. The kit for identifying tumor tissue-of-origin according to claim 1 or 2 or 3 or 4, further comprising a negative control and a positive control, wherein the negative control is a healthy human blood leukocyte DNA, and the positive control is a cancer tissue sample DNA.

7. A computer-implemented data analysis method for analyzing the data obtained by the kit for identifying tumor tissue-of-origin of claim 1, comprising the following steps:

   1) data preprocessing: conducting quality control on a raw off-machine data, and removing an adapter sequence and an inline barcode sequence of a raw data to obtain clean data;
   2) alignment: allowing the clean data aligned to the human reference genome, and converting a resulting bam file generated by the alignment into an mHap file;
   3) CpG methylation information statistics: extracting a methylation information of each CpG site;
   4) quality control: removing a sample with less than 800,000 CpG sites and having a bisulfite conversion rate of less than 99%, an alignment rate of less than 50%, and a coverage of not less than $10\times$; and
   5) analysis: analyzing a Beta value-based methylation index, predicting all samples in sequence with a training set model, and outputting a probability value of each sample on 10 cancer types.

8. The computer-implemented data analysis method according to claim 7, wherein the quality control is conducted on the raw off-machine data by a FastQC software component in step 1).

9. The computer-implemented data analysis method according to claim 8, wherein the adapter sequence and the inline barcode sequence of the raw data are removed by Trim Galore software in step 1).


## Patentansprüche

1. Ein Kit zur Identifizierung des Ursprungsgewebes von Tumoren, umfassend einen Adapter, PCR-Amplifikationsprimer und ein PCR-Amplifikationsreagenz, wobei der Adapter aus den in SEQ ID NOs: 1-12 aufgeführten Nukleotidsequenzen besteht und wobei die PCR-Amplifikationsprimer aus den in SEQ ID NOs: 13-16 aufgeführten Nukleotidsequenzen bestehen.

2. Das Kit zur Identifizierung des Ursprungsgewebes von Tumoren nach Anspruch 1, ferner umfassend ein PCR-Amplifikationsreagenz, wobei das PCR-Amplifikationsreagenz eine Polymerase, dNTP, $MgCl_2$ und Tris-HCl umfasst.

3. Das Kit zur Identifizierung des Ursprungsgewebes von Tumoren nach Anspruch 1, ferner umfassend eine Dephosphorylase und einen 10x-Puffer, wobei der 10x-Puffer ausgewählt ist aus der Gruppe bestehend aus KAc, Tris-Ac und $Mg(Ac)_2$.

4. Das Kit zur Identifizierung des Ursprungsgewebes von Tumoren nach Anspruch 1, ferner umfassend eine dNTP-Mischung, wobei die dNTP-Mischung dATP, dCTP und dGTP umfasst.

**5.** Das Kit zur Identifizierung des Ursprungsgewebes von Tumoren nach Anspruch 1 oder 2 oder 3 oder 4, ferner umfassend ein Endreparaturenzym, eine Ligase und ATP.

**6.** Das Kit zur Identifizierung des Ursprungsgewebes von Tumoren nach Anspruch 1 oder 2 oder 3 oder 4, ferner umfassend eine Negativkontrolle und eine Positivkontrolle, wobei es sich bei der Negativkontrolle um DNA aus gesunden menschlichen Blutleukozyten und bei der Positivkontrolle um DNA aus einer Krebsgewebeprobe handelt.

**7.** Ein computergestütztes Datenanalyseverfahren zur Auswertung der mit dem Kit zur Identifizierung des Tumorgewebeursprungs gemäß Anspruch 1 gewonnenen Daten, bestehend aus folgenden Schritten:

1) Datenvorverarbeitung: Durchführung einer Qualitätskontrolle der rohen, extern Maschinendaten und Entfernung einer Adaptersequenz und einer Inline-Barcodesequenz aus den Rohdaten, um bereinigte Daten zu erhalten;
2) Alignment: Ausrichtung der bereinigten Daten am menschlichen Referenzgenom und Konvertierung der resultierenden BAM-Datei durch das Alignment in eine mHap-Datei;
3) CpG-Methylierungsinformationsstatistik: Extraktion der Methylierungsinformation jeder CpG-Stelle;
4) Qualitätskontrolle: Ausschluss von Proben mit weniger als 800.000 CpG-Stellen, einer Bisulfit-Konversionsrate von unter 99%, einer Alignment-Rate von unter 50% und einer Abdeckung von mindestens $10\times$; und
5) Analyse: Analyse eines auf dem Beta-Wert basierenden Methylierungsindex, Vorhersage aller Proben nacheinander mit einem Trainingsdatensatzmodell und Ausgabe eines Wahrscheinlichkeitswertes für jede Probe in Bezug auf 10 Krebsarten.

**8.** Das computergestützte Datenanalyseverfahren nach Anspruch 7, wobei die Qualitätskontrolle der rohen, externen Maschinendaten durch eine FastQC-Softwarekomponente in Schritt 1 durchgeführt wird.

**9.** Das computergestützte Datenanalyseverfahren nach Anspruch 8, wobei die Adaptersequenz und die Inline-Barcodesequenz der Rohdaten in Schritt 1 mit der Software Trim Galore entfernt werden.

**Revendications**

**1.** Kit d'identification d'un tissu d'origine tumorale, comprenant un adaptateur, des amorces d'amplification PCR et un réactif d'amplification PCR, dans lequel l'adaptateur est constitué par les séquences nucléotidiques indiquées dans SEQ ID N° : 1-12 ; et dans lequel les amorces d'amplification PCR sont constituées par les séquences nucléotidiques indiquées dans SEQ ID N° : 13-16.

**2.** Kit d'identification d'un tissu d'origine tumorale selon la revendication 1, comprenant en outre un réactif d'amplification PCR, dans lequel le réactif d'amplification PCR comprend une polymérase, du dNTP, du $MgCl_2$ et du Tris-HCl.

**3.** Kit d'identification d'un tissu d'origine tumorale selon la revendication 1, comprenant en outre une déphosphorylase et un tampon $10\times$, dans lequel le tampon $10\times$ est choisi dans le groupe constitué par du KAc, du Tris-Ac et du $Mg(Ac)_2$.

**4.** Kit d'identification d'un tissu d'origine tumorale selon la revendication 1, comprenant en outre un mélange de dNTP, dans lequel le mélange de dNTP comprend du dATP, du dCTP et du dGTP.

**5.** Kit d'identification d'un tissu d'origine tumorale selon la revendication 1 ou 2 ou 3 ou 4, comprenant en outre une enzyme de réparation d'extrémité, une ligase et de l'ATP.

**6.** Kit d'identification d'un tissu d'origine tumorale selon la revendication 1 ou 2 ou 3 ou 4, comprenant en outre un témoin négatif et un témoin positif, dans lequel le témoin négatif est un ADN de leucocyte de sang humain sain, et le témoin positif est un échantillon d'ADN de tissu cancéreux.

**7.** Procédé d'analyse de données mis en œuvre par ordinateur pour l'analyse des données obtenues par le kit d'identification d'un tissu d'origine tumorale selon la revendication 1, comprenant les étapes suivantes :

1) pré-traitement de données : réalisation d'un contrôle de qualité sur une données machine brute, et élimination d'une séquence d'adaptateur et d'une séquence de code à barres en ligne d'une donnée brute pour obtenir des données propres ;

2) alignement : autorisation des données propres alignées sur le génome de référence humain, et conversion d'un fichier de bam résultant généré par l'alignement en un fichier mHap ;

3) statistiques d'informations de méthylation CpG : extraction d'informations de méthylation de chaque site CpG ;

4) contrôle de qualité : élimination d'un échantillon avec moins de 800 000 sites CpG et présentant un taux de conversion au bisulfite inférieur à 99 %, un taux d'alignement inférieur à 50 %, et une couverture non inférieure à 10× ; et

5) analyse : analyse d'un indice de méthylation basé sur une valeur Bêta, prédiction de tous les échantillons en séquence avec un modèle d'ensemble d'apprentissage, et délivrance en sortie d'une valeur de probabilité de chaque échantillon sur 10 types de cancer.

8. Procédé d'analyse de données mis en œuvre par ordinateur selon la revendication 7, dans lequel le contrôle de qualité est réalisé sur les données brutes de machine hors machine par le composant logiciel FastQC à l'étape 1).

9. Procédé d'analyse de données mis en œuvre par ordinateur selon la revendication 8, dans lequel la séquence d'adaptateur et la séquence de code à barres en ligne des données brutes sont éliminées par le logiciel Trim Galore à l'étape 1).

Dephosphorylation

Enzyme digestion

End repair

PCR amplification

Purification and
Bisulfite conversion

Adapter
addition

Sequencing

Data analysis

C    U

C

**FIG. 1**

EP 4 458 989 B1

**FIG. 2**

**FIG. 3**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 113249439 A
- CN 113604540 A
- WO 2008015396 A2, EARNSHAW DAVID JAMES
- EP 2898100 A1
- WO 2022159035 A1
- US 2023126920 A1, CI WEIMIN
- CN 113550013 A

**Non-patent literature cited in the description**

- **GUO SHICHENG et al.** Identification of methylation haplotype blocks aids in deconvolution of heterogeneous tissue samples and tumor tissue-of-origin mapping from plasma DNA. *NATURE GENETICS*, 06 March 2017, ISSN 1061-4036, 635-64